(19) Europäisches Patentamt
European Patent Office
Office européen des brevets

(11) **EP 2 400 880 B1**

(12) **EUROPÄISCHE PATENTSCHRIFT**

(45) Veröffentlichungstag und Bekanntmachung des Hinweises auf die Patenterteilung:
**06.11.2013   Patentblatt 2013/45**

(51) Int Cl.:
***A61B 3/113*** (2006.01)

(21) Anmeldenummer: **10704094.1**

(22) Anmeldetag: **04.02.2010**

(86) Internationale Anmeldenummer:
**PCT/EP2010/000698**

(87) Internationale Veröffentlichungsnummer:
**WO 2010/097161 (02.09.2010 Gazette 2010/35)**

(54) **VERFAHREN UND VORRICHTUNG ZUR BESTIMMUNG DER AUGENDREHPUNKTLAGE**

METHOD AND DEVICE FOR DETERMINING THE LOCATION OF THE EYE FULCRUM

PROCÉDÉ ET DISPOSITIF POUR DÉTERMINER LA POSITION DU POINT DE ROTATION DE L'OEIL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **26.02.2009   DE 102009010467**

(43) Veröffentlichungstag der Anmeldung:
**04.01.2012   Patentblatt 2012/01**

(60) Teilanmeldung:
**12002380.9 / 2 471 441**

(73) Patentinhaber: **Carl Zeiss Vision GmbH
73430 Aalen (DE)**

(72) Erfinder:
• **KRATZER, Timo
73434 Aalen (DE)**
• **CABEZA-GUILLEN, Jesus-Miguel
73434 Aalen (DE)**
• **KELCH, Gerhard
73434 Aalen (DE)**

(74) Vertreter: **Braunger, Dieter
Carl Zeiss AG
Patentabteilung
Carl-Zeiss-Straße 22
73447 Oberkochen (DE)**

(56) Entgegenhaltungen:
| | |
|---|---|
| EP-A1- 1 154 302 | EP-A1- 1 364 612 |
| EP-A1- 1 837 699 | EP-A2- 0 350 957 |
| EP-A2- 1 767 174 | WO-A1-02/09025 |
| JP-A- 2004 008 768 | US-A1- 2005 134 799 |
| US-B1- 6 241 355 | |

• THIBOS L N ET AL: "Clinical applications of the Shack-Hartmann aberrometer." OPTOMETRY AND VISION SCIENCE : OFFICIAL PUBLICATION OF THE AMERICAN ACADEMY OF OPTOMETRY DEC 1999, Bd. 76, Nr. 12, Dezember 1999 (1999-12), Seiten 817-825, XP002575578 ISSN: 1040-5488

EP 2 400 880 B1

**Beschreibung**

Die Erfindung bezieht sich auf Verfahren zur Bestimmung der Augendrehpunktlage nach dem Oberbegriff des Patentanspruchs 1.

Die Erfindung betrifft weiter Verfahren zum Optimieren eines für ein Auge individuellen Brillenglases, bei dem die Augendrehpunktlage ermittelt und als Eingangsparameter verwendet wird.

Die Offenbarung bezieht sich schließlich auf ein Verfahren zum Bestimmen von mindestens zwei optischen Parametern eines eine Referenzstruktur aufweisenden Auges, bei dem für die Bestimmung des jeweiligen Parameters das Auge jeweils mittels eines separaten Messgerätes photographisch aufgenommen wird.

Die Erfindung betrifft ferner Vorrichtungen zum Durchführen des vorstehend genannten Verfahrens.

Es ist bekannt, zum Optimieren von Brillengläsern, insbesondere von individuellen Gleitsichtgläsern, verschiedene Parameter des Systems Auge/Brille zu berücksichtigen. Diese Parameter sind beispielsweise der Pupillenabstand PD, der Hornhaut-Scheitelabstand HSA, der Vorneigungswinkel $\alpha$ des Brillenglases, die Fassungsscheibenwinkel $\alpha_R$, $\alpha_L$ für das rechte und linke Brillenglas sowie die Lage des optischen oder mechanischen Augendrehpunkts Z', M. Unter individuellen Gleitsichtgläsern versteht man Gleitsichtgläser, bei denen mindestens ein individueller Gebrauchsparameter bei der Berechnung des Brillenglases berücksichtigt wird und die mittels Freiformtechnologie gefertigt werden, wie dies z.B. in der Deutschen Optikerzeitung DOZ 4 + 5/2000 von W. Grimm und G. Kelch in dem Aufsatz "Gradal® Individual: Konzeption, Fertigung und Anpassung", in der EP 857 993 B1 und/oder der EP 562 336 B1 beschrieben ist.

Bei nicht-individuellen Brillengläsern werden diese Parameter aus statistischen Mittelwerten eines repräsentativen Bevölkerungsquerschnitts ermittelt. Bei individuellen Brillengläsern hingegen werden die Parameter individuell an dem jeweiligen Brillenträger gemessen,  beispielsweise mit so genannten Videozentriersystemen, wie sie von der Anmelderin unter den Typenbezeichnungen "i.Terminal" und "Relaxed Vision Terminal" hergestellt und vertrieben werden. Diese Videozentriersysteme sind jedoch bisher nicht in der Lage, eine Optimierung des Brillenglases mit Hilfe der Augendrehpunktlage in einer ausreichend exakten Weise zu ermöglichen.

Wenn man die Augendrehpunktlage berücksichtigen möchte, kann man diese in bekannter Weise aus der Augenlänge über die mittlere Sphäre des verordneten Brillenglases ableiten. Dabei werden allerdings viele Annahmen gemacht, die mit den tatsächlichen Gegebenheiten nicht ausreichend übereinstimmen.

Der Zusammenhang zwischen Augenlänge und der Sphäre des verordneten Brillenglases wird oft vereinfacht als linear angenommen. Dies ist aber in der Realität nicht der Fall, weil sowohl die Krümmung der Hornhaut als auch die Augenlinse sowie die Augenlänge teilweise sehr unabhängig voneinander wachsen bzw. sich unterschiedlich entwickeln.

Im Allgemeinen ist es ausreichend, den Augendrehpunkt als punktförmiges Drehzentrum innerhalb des Auges zu betrachten. Die Erfindung umfasst jedoch ganz allgemein auch ein ausgedehntes, im Allgemeinen näherungsweise kugelförmiges Augendrehpunktsgebiet. Zur Beschreibung der Erfindung und des Standes der Technik wird nachfolgend der Einfachheit halber von einem punktförmigen Augendrehpunkt ausgegangen, soweit nichts Anderes erwähnt ist.

Üblicherweise geht man bei der Verordnung einer Brille so vor, dass die Brillengläser auf der Grundlage einer subjektiven Refraktion sowie einer Videozentriermessung verordnet und zentriert werden.

Dabei ist von Nachteil, dass es keine Referenzierung zwischen der Refraktionsbestimmung und der Zentrierung gibt, weil beide Vorgänge mit unterschiedlichen Apparaturen durchgeführt werden. Wenn die Refraktion beispielsweise mit einem Phoropter ermittelt wird, kann es vorkommen, dass der Phoropter um einen ersten Winkel relativ zu der der Linie verkippt ist, die die beiden Pupillenmittelpunkte des Auges verbindet. Ferner kann es vorkommen, dass die gewählte Brillenfassung relativ zu dieser Linie um einen zweiten Winkel verdreht ist. Im ungünstigsten Falle können sich die beiden Winkel addieren, was zu  einer Diskrepanz von mehreren Winkelgraden in den Achsen der Zylinder zwischen der Verordnung und der ausgeführten Korrektur der fertigen Brille führen kann.

Aus der WO 2006/106248 A1 sind ein Verfahren und eine Vorrichtung zum Bestimmen der Augendrehpunktlage bekannt. Bei diesem bekannten Verfahren blickt ein Proband in ein fernrohrartiges Gerät. Die Ausrichtung des Gerätes im Raum wird durch einen am Gerät befindlichen, dreidimensional wirksamen Sensor bestimmt. Der Proband trägt an seinem Kopf einen weiteren derartigen Sensor, der die Lage und Ausrichtung seines Kopfes bestimmt. In dem Gerät befindet sich auf der vom Probanden abgewandten Seite eine Lichtquelle, die einen Lichtstrahl längs einer optischen Achse ausstrahlt. Zwischen der Lichtquelle und dem Auge des Probanden befinden sich zwei Gitter mit zentralen Marken. Der Proband bewegt das Gerät nun so lange, bis sich der Lichtstrahl und die beiden Marken decken. Aus den dabei ermittelten Positionsdaten für das Gerät und den Kopf wird die Lage der Blickachse im Raum errechnet. Der Vorgang wird dann mehrfach unter unterschiedlichen Blickrichtungen wiederholt, so dass mehrere Blickachsen bestimmt werden. Deren Drehpunkt wird dann als Augendrehpunktlage berechnet.

Diese bekannte Vorgehensweise hat den Nachteil, dass ein erheblicher Aufwand an separaten Geräten betrieben werden muss. Ferner ist die Messgenauigkeit vom subjektiven Verhalten des Probanden abhängig.

Aus der EP 0 825 826 A1 sind ein Verfahren und eine Vorrichtung zum parallelen Erfassen von Sehinformationen bekannt. Zum Bestimmen der Augendrehpunktlage wird bei dieser bekannten Vorgehensweise bei fester Ausrichtung und Lage des Kopfes des Probanden mindestens für zwei bekannte, nacheinander von den Augen fixierte Fixierpunkte eine Fixierlinien-Bestimmung durchgeführt. Die Fixierpunkte sind zwei fest mit der Halterung von Kameras und Sensoren zur Bestimmung der Pupillenlage verbindbare Markierpunkte. Der Augendrehpunkt liegt dann im Schnittpunkt der durch die Fixierpunkte gelegten Fixierlinien.

Der Erfindung liegt die Aufgabe zugrunde, andere Verfahren zur Bestimmung der Augendrehpunktlage zur Verfügung zu stellen. Ferner soll ein Verfahren bereitgestellt werden, das es möglich macht, beim Erfassen von Parametern des Systems Auge/Brille mit mehreren verschiedenen Messungen, insbesondere unter Verwendung von unterschiedlichen Geräten, eine gemeinsame räumliche Referenz für die Messwerte zu finden.

Bei einem ersten der eingangs genannten Verfahren wird diese Aufgabe gelöst durch die Schritte:

a) Ermitteln der mittleren Krümmung der Hornhaut des Auges;

b) Ermitteln des mittleren Phasenfehlers des Auges;

c) Ermitteln der Augenlänge aus der mittleren Krümmung und dem mittleren Phasenfehler; und

d) Bestimmen der Augendrehpunktlage aus der Augenlänge.

Die mittlere Krümmung der Hornhaut bezeichnet den Mittelwert der Krümmung der Hornhaut im Bereich des Hornhautscheitels, im Allgemeinen in einem kreisförmigen Bereich mit dem Radius 4 mm um den Hornhautscheitel. Der Phasenfehler des Auges ist die Abweichung der Phase einer aus dem Auge austretenden Wellenfront von einer Referenzwelle, hier i.A. von einer ebenen Wellenfront. Der mittlere Phasenfehler bezeichnet die mittlere Krümmung der Wellenfront. Die Augenlänge ist die geometrische Länge des Auges zwischen Hornhautscheitel und der Fovea. Unter Augendrehpunktlage versteht man ganz allgemein den Ort des optischen Augendrehpunkts. Als optischer Augendrehpunkt (Kurzzeichen Z') wird z.B. nach der DIN 5340-43 der Fußpunkt des Lotes vom mechanischen Augendrehpunkt auf die ins Augeninnere verlängerte Fixierlinie beim Blick geradeaus auf einen unendlich fernen Punkt bei ungezwungener Kopf- und Körperhaltung angenommen. Der mechanische Augendrehpunkt (Kurzzeichen M) ist z.B. nach der DIN 5340-42 derjenige Punkt im Auge, der sich bei Blickbewegungen am wenigsten verlagert.

Bei einem anderen zweiten der eingangs genannten Verfahren wird diese Aufgabe gelöst durch die Schritte:

a) Erfassen der Lage und/oder der Form eines charakteristischen Bestandteils des Auges bei mindestens zwei beliebigen Blickrichtungen, d.h. im Unterschied zur Offenbarung der EP 0 825 826 A1 nicht fest vorgegebenen Markierungen und/oder nicht festgelegter Lage des Kopfes;

b) Ermitteln der Lage wenigstens einer charakteristischen Achse des Auges für die zwei Blickrichtungen aus der jeweiligen Lage und/oder der jeweiligen Form des charakteristischen Bestandteils des Auges bei den beiden Blickrichtungen;

c) Bestimmen der Augendrehpunktlage aus der Lage der wenigstens einen charakteristischen Achse des Auges für die zwei Blickrichtungen.

Bei einem weiteren dritten der eingangs genannten Verfahren wird diese Aufgabe erfindungsgemäß gelöst durch die Schritte:

a) dreidimensionales Messen wenigstens eines Teils der Oberfläche der Hornhaut des Auges bei einer ersten Blickrichtung des Probanden;

b) Beschreiben der in Schritt a) ermittelten Oberfläche der Hornhaut durch eine dreidimensionale mathematische Formel;

c) dreidimensionales Messen mindestens eines Teils der Oberfläche der Hornhaut bei gegenüber der Blickrichtung in Schritt a) geänderter Blickrichtung des Probanden in derselben Messposition wie bei Schritt a), bei gegenüber Schritt a) unveränderter Kopfposition des Probanden;

d) Bestimmen der Blickrichtung in Schritt c) und/oder der Augendrehpunktlage durch Fitten der in Schritt c) gemessenen dreidimensionalen Daten an die in Schritt b) ermittelte Formel, wobei eine mathematische Transformation, insbesondere Drehung um einen Punkt im Raum, verwendet wird.

Bei einem vierten der eingangs genannten Verfahren wird diese Aufgabe dadurch gelöst, dass bei den photographischen Aufnahmen jeweils zugleich die Referenzstruktur des Auges aufgenommen wird und dass die Werte der Parameter auf die Referenzstruktur bezogen werden.

Die der Erfindung zugrunde liegende Aufgabe wird ferner durch Vorrichtungen zum Durchführen der vorstehend genannten Verfahren gelöst.

Insbesondere ist eine erste der eingangs genannten Vorrichtungen zur Bestimmung der Augendrehpunktlage in einem Auge eines Probanden gekennzeichnet durch:

a) eine Krümmungsermittlungseinrichtung zum Ermitteln der mittleren Krümmung der Hornhaut des Auges;

b) eine Phasenfehlermesseinrichtung zum Ermitteln des mittleren Phasenfehlers des Auges;

c) eine Augenlängenberechnungseinrichtung zum Ermitteln der Augenlänge aus der mittleren Krümmung und dem mittleren Phasenfehler; und

d) eine Augendrehpunktbestimmungseinrichtung zum Bestimmen der Augendrehpunktlage aus der Augenlänge.

Die Krümmungsermittlungseinrichtung kann z.B. ein Videokeratograph oder ein Keratometer sein. Die Phasenfehlermesseinrichtung kann z.B. als Autorefraktor oder Wellenfrontsensor ausgebildet sein. Die Augenlängenberechnungseinrichtung und die Augendrehpunktbestimmungseinrichtung können z.B. gemeinsam in Form eines handelsüblichen Personal Computers realisiert sein.

Eine andere zweite der eingangs genannten Vorrichtungen ist gekennzeichnet durch:

a) eine Aufnahmeeinrichtung zum Erfassen der Lage und/oder der Form eines charakteristischen Bestandteils des Auges bei mindestens zwei beliebigen Blickrichtungen im vorstehend beschriebenen Sinn;

b) eine Ermittlungseinrichtung zum Ermitteln der Lage wenigstens einer charakteristischen Achse des Auges für die zwei Blickrichtungen aus der Lage und/oder der Form des charakteristischen Bestandteils des Auges;

c) eine Bestimmungseinrichtung zum Bestimmen der Augendrehpunktlage aus der Lage der charakteristischen Achse des Auges für die zwei Blickrichtungen.

Die Aufnahmeeinrichtung kann z.B. eine Zentriereinheit oder eine Digitalkamera sein. Die Ermittlungseinrichtung und die Augendrehpunkt-Bestimmungseinrichtung können z.B. gemeinsam in Form eines handelsüblichen Personal Computers realisiert sein.

Eine weitere dritte der eingangs genannten Vorrichtungen umfasst erfindungsgemäß:

a) eine Messeinrichtung zum dreidimensionales Messen wenigstens eines Teils der Oberfläche der Hornhaut des Auges bei einer ersten Blickrichtung des Probanden und zum dreidimensionales Messen mindestens eines Teils der Oberfläche der Hornhaut bei gegenüber der ersten Blickrichtung geänderter zweiter Blickrichtung des Probanden in derselben Messposition und bei unveränderter Kopfposition des Probanden; und

b) eine Recheneinrichtung zum Beschreiben der zur ersten Blickrichtung ermittelten Oberfläche der Hornhaut durch eine dreidimensionale mathematische Formel und zum Bestimmen der zweiten Blickrichtung sowie der Augendrehpunktlage durch Fitten der zur zweiten Blickrichtung gemessenen dreidimensionalen Daten an die zur ersten Blickrichtung ermittelte Formel, wobei eine mathematische Transformation, insbesondere Drehung um einen Punkt im Raum, verwendet wird.

Als Messeinrichtung kann z.B. ein Videokeratograph verwendet werden. Die Recheneinrichtung kann z.B. ein handelsüblicher Personalcomputer sein.

Schließlich ist eine vierte Vorrichtung zum Bestimmen von mindestens zwei optischen Parametern eines eine Referenzstruktur aufweisenden Auges, mit separaten Aufnahmegeräten zum photographischen Aufnehmen des Auges für die Bestimmung jeweils eines der optischen Parameter das Auge vorgesehen. Die Aufnahmegeräte sind derart ausgebildet, dass bei den photographischen Aufnahmen jeweils zugleich die Referenzstruktur des Auges aufgenommen wird. Es ist (zumindest) eine Recheneinrichtung vorgesehen, um die Werte der Parameter auf die Referenzstruktur zu beziehen.

Die der Erfindung zugrunde liegende Aufgabe wird durch die in den unabhängigen Ansprüchen definierten Merkmalen vollkommen gelöst.

Die ersten drei Verfahren zeichnen sich dadurch aus, dass die Augendrehpunktlage noch exakter als herkömmlich bestimmt werden kann. Dadurch ist eine noch bessere Optimierung insbesondere von individuellen Gleitsichtgläsern möglich, wenn man die Augendrehpunktlage bei der Berechnung und Herstellung der Oberflächentopographie der individuellen Gleitsichtgläser berücksichtigt.

Die mittlere Krümmung der Hornhaut und der mittlere Phasenfehler des Auges können ohne Einschränkung an den Durchstoßpunkten einer gegebenen Achse mit den dreidimensionalen Messungen der Hornhauttopographie bzw. Wellenfrontfehler des Auges ermittelt werden.

Durch das erste bis dritte Verfahren wird es möglich, die vorstehend genannte noch bessere Optimierung mittels einer nur geringfügigen Modifizierung bekannter Systeme zu erreichen.

Durch das vierte Verfahren wird eine bessere Referenzierung zweier mit unterschiedlichen Geräten aufgenommener Parameter möglich und damit Fehlanpassungen einer Brille vermieden.

Bei dem zuerst genannten Verfahren kann die Augendrehpunktlage ADL aus der Augenlänge LA nach der Beziehung:

$$ADL = k_3 LA \tag{1}$$

bestimmt werden. Die in Meter angegebene Größe ADL gibt dabei den Abstand zwischen dem auf der Fixierlinie liegenden Hornhautscheitel und dem Zentrum des optischen Augendrehpunkts Z' an. $k_3$ ist ein vorgebbarer, dimensionsloser Parameter.

Dieser Parameter $k_3$ kann z.B. der Theorie von Gullstrand folgend zu 13, 5/23, 8 gewählt werden. Abweichungen von diesem Wert von z.B. $\pm$ 10% bzw. 5% können jedoch im Allgemeinen zugelassen werden.

Bei diesem zuerst genannten Verfahren kann die Augenlänge LA z.B. aus der mittleren Krümmung KH und dem mittleren Phasenfehler PF nach der Beziehung:

$$LA = (k_1 - PF) KH / k_2 \tag{2}$$

bestimmt werden. Die mittlere Krümmung KH wird im Allgemeinen in Millimeter und der mittlere Phasenfehler wird im Allgemeinen in der Dimension Dioptrien angegeben. Die Parameter $k_1$ und $k_2$ sind grundsätzlich frei vorgebbar. $k_1$ hat die Dimension des Phasenfehlers, nämlich Dioptrien, $k_2$ hat ebenfalls die Dimension Dioptrien.

Folgt man einer Theorie der Erfinder so wählt man $k_1$ zu 52, 634 dpt und $k_2$ zu 17, 395 dpt Abweichungen von diesen Werten um $\pm$ 10% bzw. $\pm$ 5% können jedoch i. A. zugelassen werden. Diese Maßnahme hat den Vorteil, dass für den bei weitem überwiegenden Anteil der Bevölkerung eine optimale Ermittlung der Augendrehpunktlage gewährleistet ist.

In diesem erstgenannten Verfahren ist es im Allgemeinen ausreichend in Schritt a) die mittlere Krümmung KH der Hornhaut im Bereich der Pupillenöffnung zu ermitteln. Da die Aufweitung der Pupillenöffnung von der Umgebungshelligkeit abhängt, nimmt man üblicherweise den Bereich im Durchmesser von 8 mm um die Pupillenmitte.

Da es bei der Krümmung KH der Hornhaut und dem Phasenfehler PF des Auges besonders auf den Bereich um die optische Achse ankommt, wird im Allgemeinen in Schritt a) die mittlere Krümmung KH der Hornhaut und in Schritt b) der Phasenfehler PF jeweils im Bereich um die optische Achse und/oder im Bereich um die Fixierlinie und/oder im Bereich um die Sehachse des Auges ermittelt werden. Typische Radien um diese Achsen sind 4 mm. Es genügen im Allgemeinen auch Radien von bis zu 2 mm oder gar 1 mm. Als optische Achse des Auges kann z.B. die in DIN 5340-vorgeschlagene Normale auf die Hornhautvorderfläche angenommen werden, deren Verlängerung in das Augeninnere von den Krümmungsmittelpunkten der übrigen brechenden Flächen des Auges den geringsten Abstand hat. Als Sehachse (Gesichtslinie) kann die nach DIN 5340- 360 vorgeschlagene Verbindungsgerade zwischen dem zentral abgebildeten Objektpunkt und seinem Bildpunkt auf der Netzhaut verwendet werden. Als Fixierlinie kann z.B. die in DIN 5340- 159 vorgeschlagene Verbindungsgerade zwischen dem in der Fovelamitte abgebildeten Objektpunkt und der Mitte der Eintrittspupille des Auges verwendet werden.

Diese vorstehend angegebenen Maßnahmen haben den Vorteil, dass die Ermittlung der genannten Werte verbessert wird, indem diese Werte zueinander räumlich referenziert ermittelt werden, also bezogen auf eine bestimmte Achse oder die Pupillenmitte.

Der Phasenfehler kann z.B. mittels eines Wellenfront-Autorefraktors nach dem Hartmann-Shack-Verfahren gemessen werden. Aus der mit Hilfe des Wellenfront-Autorefraktors gemessenen Phasenfehlerverteilung wird dann ein Mittelwert gebildet. Dieser Mittelwert stellt den mittleren Phasenfehler PF dar. Die Bestimmung des Phasenfehlers mit Hilfe eines Wellenfront-Autorefraktors hat den Vorteil, dass örtliche Variationen des Phasenfehlers berücksichtigt werden. Anstelle eines Wellenfront-Autorefraktors kann auch ein Autorefraktometer verwendet werden.

Bei dem zweiten Verfahren kann der charakteristische Bestandteil des Auges z.B. die Pupille und/oder den Limbus und/oder die Iris umfassen. Diese charakteristischen Bestandteile des Auges sind mit bloßem Auge erkennbar und können daher in einfacher Weise sowohl von einem Benutzer als auch von einem automatischen Erfassungssystem eindeutig identifiziert werden. Eine Fehlinterpretation ist daher weitgehend ausgeschlossen.

Anstelle der vorstehend angegebenen charakteristischen Augenbestandteile Pupille, Limbus und/oder Iris können selbstverständlich auch andere für das Auge charakteristische (insbesondere biometrische) Bestandteile, wie z.B. Äderchen oder in der Farbe unterscheidbare Bereiche, verwendet werden. Derartige Strukturen können sogar im Einzelfall bevorzugt sein, z.B. wenn es auf die fehlende Invarianz der Struktur gegenüber Drehungen ankommt.

Die Lage und/oder die Form des charakteristischen Bestandteils des Auges kann z.B. durch ein kalibriertes photographisches System erfasst werden. Unter kalibriertem photographischem System versteht man ein photographisches System welches zur Erfassung von dreidimensionalen Parametern des Kopf-Auge-Systems verwendet werden kann. Die Verwendung eines derartigen Systems hat den Vorteil, dass man damit mit ausreichender Genauigkeit messen kann.

Als kalibriertes photographisches System kann z.B. ein kalibriertes Videozentriersystem verwendet werden. Ein Videozentriersystem ohne Kalibrierung ist i.A. nur eine Digitalkamera und somit wertlos bei der Messung von Gebrauchsparametern.

Die wenigstens eine charakteristische Achse, deren Lage für die zwei Blickrichtungen aus der jeweiligen Lage und/oder der jeweiligen Form des charakteristischen Bestandteils des Auges bei den beiden Blickrichtungen ermittelt wird, kann z.B. die Fixierlinie und/oder die Sehachse und/oder die optischen Achse umfassen. Alle drei charakteristischen können z.B. nach dem o.a. Verfahren durch einfache Rechenoperationen aus den zuvor aufgenommenen Daten bestimmt werden.

Bei diesem zweiten Verfahren kann die Bestimmung der Augendrehpunktlage eine Bestimmung des Schnittorts der charakteristischen Achsen des Auges umfassen.

Dieses zweite Verfahren kann z.B. folgende Schritte umfassen:

a) Erfassen des charakteristischen Bestandteils des Auges und Ermitteln dessen geometrischen Mittelpunktes sowie der Normalen in der Ebene des charakteristischen Bestandteils in dem Mittelpunkt bei einer ersten Blickrichtung des Probanden;

b) Erfassen des charakteristischen Bestandteils des Auges und Ermitteln dessen geometrischen Mittelpunktes sowie der Normalen in der Ebene des charakteristischen Bestandteils in dem Mittelpunkt bei einer zweiten von der ersten Blickrichtung abweichenden Blickrichtung des Probanden; und

c) Bestimmen der Augendrehpunktlage aus den Richtungsvektoren der in den Schritten a) und b) ermittelten Normalen.

Anstelle des Schnittorts einer charakteristischen Achse bei unterschiedlichen Blickrichtungen kann man auch zwei charakteristische Achsen ermitteln und die Augendrehpunktlage als einen Schnittpunkt der beiden charakteristischen Achsen bestimmen.

Anstelle des Schnittorts zweier charakteristischer Achsen können auch mehr als zwei charakteristische Achsen ermittelt werden und die Augendrehpunktlage als Mittelpunkt eines von den charakteristischen Achsen tangential umschlossenen Kugelvolumens bestimmt werden.

Ein Verfahren zum Optimieren eines für ein Auge eines Probanden individuellen Brillenglases, bei dem die Augendrehpunktlage ermittelt und als Eingangsparameter verwendet wird, kann sich zur Bestimmung der Augendrehpunktlage in dem Auge des Probanden eines der vorstehend beschriebenen Verfahren bedienen.

Das vierte Verfahren kann z.B. die folgenden Schritte umfassen:

a) Messen einer Referenzstruktur des Auges in einer ersten Messsituation;

b) Messen der Referenzstruktur des Auges in einer zweiten Messsituation;

c) Ermitteln der Lageänderung der Referenzstruktur zwischen den beiden Messsituationen;

d) Korrigieren von verordneten Brillengläsern in Abhängigkeit von der Lageänderung

Dieses Verfahren zeichnet sich dadurch aus, dass man die Messungen nach a) und b) aufeinander referenzieren kann. Die Verfahrensschritte a) und b) können z.B. von den beiden Aufnahmeeinrichtungen durchgeführt werden. Die Verfahrensschritte c) und d) können z.B. von der Recheneinrichtung (z.B. ein Personalcomputer) ausgeführt werden.

Bei Weiterbildungen dieses Verfahrens wird zum Messen der Referenzstruktur z.B. die Lage der Pupillenmitte des Auges und/oder die Lage des Hornhautscheitels ermittelt. Die Lage der Pupillenmitte oder die Lage des Hornhautscheitels eignen sich besonders als Referenzstrukturen, weil sie einfach zu erfassen sind.

Als Referenzstrukturen eignen sich z.B. auch eine Struktur der Iris oder Blutgefässe der Lederhaut. Derartige Referenzstrukturen sind im Allgemeinen ohne jegliche Symmetrie. Sie erlauben daher eine eindeutige Lokalisierung im Raum.

Ein Computerprogramm mit Programmcode kann zur Durchführung eines der vorstehend beschriebenen Verfahren eingerichtet sein, wenn das Programm in einem Computer ausgeführt wird. Das Computerprogramm kann z.B. auf einem maschinenlesbaren Datenträger gespeichert sein.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Figur 1    eine erste schematische Seitenansicht eines nach oben verdrehten Auges zur Erläuterung von verschiedenen Blickrichtungen und Drehpunkten und der Lage des Augendrehpunktes;

Figur 2    eine zweite schematische Seitenansicht eines geradeaus gerichteten Auges mit vorgesetztem Brillenglas zur Erläuterung weiterer Parameter des Auges;

Figur 3    eine Frontansicht eines Auges zur Erläuterung bestimmter Augenbereiche;

Figur 4    eine dritte schematische Seitenansicht eines geradeaus gerichteten Auges zur Erläuterung der Messung des Phasenfehlers des Auges mit Hilfe eines Wel lenfront-Autorefraktors;

Figur 5    ein Diagramm, wie es im Rahmen des ersten Verfahrens der vorliegenden Erfindung zur Ermittlung der Augendrehpunktlage verwendet werden kann;

Figur 6    eine Darstellung einer punktweisen Vermessung der Hornhaut bei einem geradeaus gerichteten Blick des Probanden;

Figur 7    eine Darstellung der dreidimensionalen mathematischen Funktion zur Beschreibung der Hornhaut von Figur 6;

Figur 8    eine Darstellung einer punktweisen Vermessung der Hornhaut bei einem anders als in Figur 6 gerichteten Blick des Probanden;

Figur 9    eine Darstellung der Hornhautpunkte bei der Blickrichtung gemäß Figur 8 im Vergleich zur Blickrichtung gemäß Figur 6; und

Figur 10   eine Darstellung des Ergebnisses nach einem Fitten der Hornhautpunkte gemäß Figur 9 an die mathematische Funktion gemäß Figur 7;

Figur 11   eine Vorrichtung zur Bestimmung der Augendrehpunktlage in einem Auge eines Probanden nach der Erfindung;

Figur 12   eine andere Vorrichtung zur Bestimmung der Augendrehpunktlage nach der Erfindung;

Figur 13   eine weitere Vorrichtung zur Bestimmung der Augendrehpunktlage nach der Erfindung;

Figur 14   eine Vorrichtung zum Bestimmen von mindestens zwei optischen Parametern eines Auges nach der Erfindung.

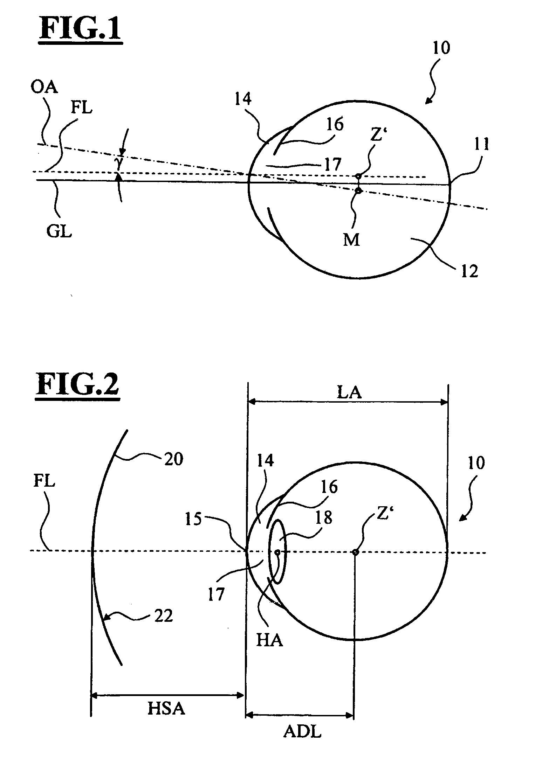

In den Figuren 1 und 2 bezeichnet 10 ein Auge. Das Auge 10 hat einen Glaskörper 12, eine Hornhaut 14, eine Iris 16, eine Pupille 17 sowie eine Linse 18.

Wenn das Auge 10 eine Drehbewegung ausführt, dann geschieht dies nicht exakt um einen Drehpunkt im Raum. Vielmehr gibt es nur einen näherungsweise kugelförmigen Bereich, in dem sich die momentanen Drehpunkte befinden. Derjenige Punkt, der bei Augenbewegungen die geringste Lageveränderung erfährt, wird als mechanischer Augendrehpunkt M bezeichnet (vgl. DIN 5340- 42).

Mit GL ist die Sehachse (Gesichtslinie) bezeichnet. Sie ist nach DIN 5340- 360 die Verbindungsgerade zwischen einem fixierten Objektpunkt und dem dazu konjugierten Bildpunkt in der Mitte der Netzhautgrube 11.

Mit FL ist die Fixierlinie (Visierlinie) bezeichnet. Sie ist nach DIN 5340- 159 die Verbindungsgerade zwischen dem zentral abgebildeten Objektpunkt und der Mitte der Eintrittspupille 17.

OA bezeichnet die optische Achse.

Mit Z' ist der optische Augendrehpunkt bezeichnet. Er ist nach DIN 5340- 43 der Fußpunkt des Lotes vom mechanischen Augendrehpunkt M auf die Fixierlinie FL.

Der Winkel zwischen der optischen Achse OA und der Fixierlinie FL ist in Figur 1 mit $\gamma$ bezeichnet. Der Winkel $\gamma$ ist hier nur in einer Ebene eingezeichnet, symbolisiert aber einen Raumwinkel oben/unten und /rechts/links.

In Figur 2 ist ein Brillenglas 20 vor dem Auge 10 angeordnet. Das Brillenglas hat auf der dem Auge 10 zugewandten Seite eine Rückfläche 22. Der Abstand der Rückfläche 22 von dem Hornhaut-Apex 15 gemessen in Blickrichtung senkrecht zur Fassungsebene wird als Hornhaut-Scheitelabstand HSA bezeichnet (vgl. DIN EN ISO 13666- 5.27). Der Abstand des Hornhautscheitels 15 vom optischen Augendrehpunkt Z' gibt die Augendrehpunktlage ADL in Bezug auf den Hornhaut-Apex 15 an.

Die Augendrehpunktlage ADL ist ein wichtiger Parameter in der Berechnung des Brillenglases 20. Das Brillenglas 20 wird immer so optimiert, dass es für jede Blickrichtung des Auges 10 die optimalen Abbildungseigenschaften aufweist.

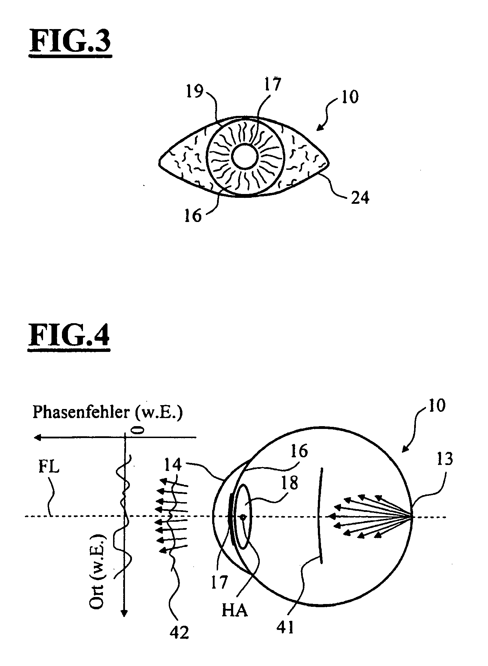

Figur 3 zeigt eine Frontansicht des Auges 10. Man erkennt in der Iris 17 eine charakteristische Struktur sowie neben der Iris 17 in der Lederhaut 24 eine Struktur von kleinen Blutgefäßen.

Erstes Ausführungsbeispiel:

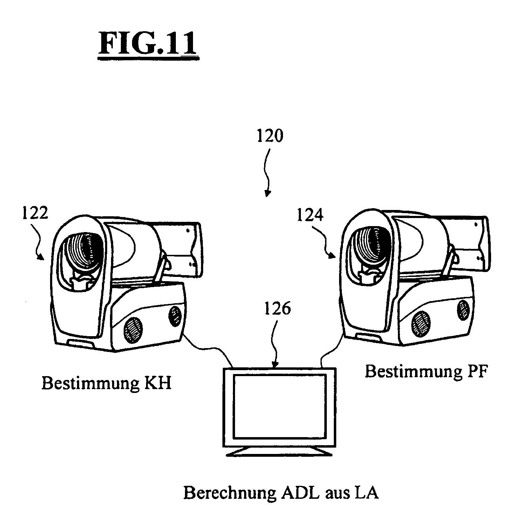

Bei einem ersten Ausführungsbeispiel eines Verfahrens wird die Augendrehpunktlage ADL auf der Grundlage eines Augenmodells und einer Abschätzung der Augenlänge LA ermittelt. Eine erfindungsgemäße Vorrichtung 120 zur Durchführung des Verfahrens zeigt die Figur 11. Das Verfahren umfasst folgende Schritte:

In einem ersten Schritt 1a) wird mit einem Gerät 122, nämlich mittels eines geeigneten Scanners die Topographie der Hornhaut 14 und daraus die mittlere Krümmung KH der Hornhaut 14 ermittelt. Bei dieser Messung kann zugleich die Lage der Mitte der Pupille 17 sowie zusätzlich die Lage des Scheitels der Hornhaut 14 ermittelt werden.

In einem zweiten Schritt 1b) werden die Phasenfehler und der Mittelwert des Phasenfehlers PF des Auges 10 ermittelt. Hierzu bedient man sich beispielsweise eines Wellenfront-Autorefraktors 124 bekannter Bauart, der die Verteilung der Phasenfehler über die gesamte Öffnung der Pupille 17 des Auges 10 ermittelt, wie dies in der Figur 4 dargestellt ist. Die Phasenfehlerverteilung kann Beispielsweise mit dem sogenannten Standard Hartmann-Shack-Verfahren bestimmt werden. Dieses Verfahren basiert auf dem Vergleich einer an der Netzhaut 13 gestreuten und durch das Auge hindurch getretenen Wellenfront 42 mit der Wellenfront 41 vor der Streuung und dem Augendurchtritt. Dabei kann auch die Lage der Mitte der Pupille 17 ermittelt werden. Die beiden Messungen können ggf. auch mit zwei unterschiedlichen Messanordnungen in einem Gerät durchgeführt werden (aus diesem Grund zeigt die Figur 11 zwei identische Geräte 122, 124). Ein Computer 126 errechnet dann aus der Verteilung der Phasenfehler über dem Ort den arithmetischen Mittelwert des Phasenfehlers PF des Auges 10 des Probanden.

Um in den vorgenannten beiden Schritten ein gemeinsames Bezugssystem für die gemessenen Lagen herzustellen, kann zusätzlich in jedem der beiden Schritte 1a) und 1b) eine photographische Aufnahme hergestellt werden, die beispielsweise die in Figur 3 dargestellten Strukturen der Iris 17 oder der Blutgefäße in der Lederhaut 24 erfasst. Diese Strukturen können dann als Referenzsystem für die Lagen der Pupillenmitte und des Hornhautscheitels sowie weiterer Parameter verwendet werden.

Aus den so ermittelten Werten KH und PF wird nun in einem dritten Schritt 1c) die Augenlänge LA bestimmt. Hierzu kann man sich der Beziehung

$$LA = (k_1 - KR)KH/k_2$$

bedienen, deren Konstanten $k_1$ und $k_2$ aus einem Modell gewonnen werden, das durch Messungen an einer Vielzahl von Augen erstellt wird/worden ist.

Figur 4 zeigt hierzu ein Diagramm 30 mit dem Zusammenhang zwischen dem Quotienten aus Augenlänge und Hornhautkrümmung (Abszisse LA/KH) und des Phasenfehlers des Auges (Ordinate PF). Die Punkte bezeichnen die Ergebnisse realer Messungen an Testpersonen. Die Ausgleichsgerade 34 gibt den linearen Zusammenhang zwischen den Messwerten wieder.

Aus dem Verlauf der Geraden 34 kann man die Konstanten $k_1$ und $k_2$ wie folgt entnehmen:

$$k_1 = 52,634 \text{ dpt}$$

$$k_2 = 17,395 \text{ dpt}$$

Mit der so bestimmten Augenlänge LA kann man in einem vierten Schritt 1d) die Augendrehpunktlage ADL nach der Beziehung

$$ADL = k_3 LA$$

ermitteln, wobei z.B. nach Gullstrand als Erfahrungswert gilt:

$$k_3 = 13,5/23,8$$

Die derart bestimmte Augendrehpunktlage ADL kann als Eingangsparameter in der Berechnung eines individuell optimierten Brillenglases verwendet werden.

Bevorzugt wird in Schritt a) die mittlere Krümmung KH der Hornhaut 14 im Bereich der Pupillenöffnung ermittelt. Alternativ oder zusätzlich kann in Schritt a) die mittlere Krümmung KH der Hornhaut 14 und in Schritt b) der Phasenfehler PF jeweils im Bereich um eine Achse OA, GL des Auges 10 ermittelt werden. Die mittlere Krümmung KH der Hornhaut 14 wird dabei insbesondere in einem Bereich mit dem Durchmesser 12 mm um den Hornhautscheitel ermittelt, der mittlere Phasenfehler PF in einem entsprechenden Bereich um die Pupillenmitte. Die beiden Werte werden auf diese Weise räumlich referenziert, also z.B. beide auf eine bestimmte charakteristische Achse OA oder GL oder die Pupillenmitte bezogen.

Zweites Ausführungsbeispiel:

Gemäß einem zweiten Ausführungsbeispiel eines Verfahrens ist es auch möglich, Lage und/oder der Form eines charakteristischen Bestandteils des Auges bei mindestens zwei Blickrichtungen zu erfassen und aus diesen wiederum die Lage wenigstens einer charakteristischen Achse des Auges für diese zwei Blickrichtungen zu ermitteln und anhand dieser charakteristischen Achse(n) des Auges für die beiden Blickrichtungen die Augendrehpunktlage zu bestimmen.

Die derart bestimmte Augendrehpunktlage kann dann wiederum als Eingangsparameter in der Berechnung eines individuell optimierten Brillenglases verwendet werden.

Die erste Variante, nämlich die Bestimmung der Augendrehpunktlage aus der Lage eines charakteristischen Bestandteils des Auges, ergibt sich wie folgt:

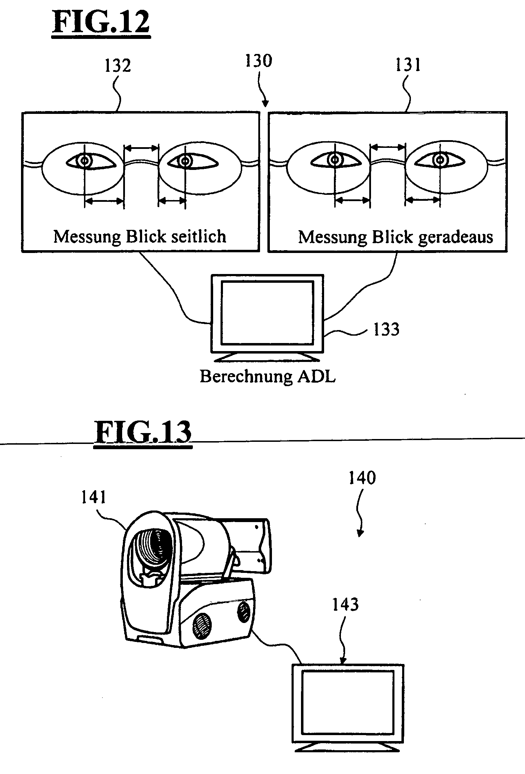

Dem Probanden wird aufgetragen, ein bestimmtes Fixierziel anzusehen. Es erfolgt eine Aufnahme des Auges für diese Blickrichtung mit Hilfe eines kalibrierten photographischen Systems. Ein derartiges kalibriertes photographisches System kann ein Video-Zentriersystem sein, wie es von der Anmelderin unter den Bezeichnungen RVT und i.Terminal vertrieben wird. Zu diesem Zweck muss das Video-Zentriersystem lediglich so eingesetzt werden, dass es Bilder des Auges 10 in unterschiedlichen Blickrichtungen aufnehmen kann. Das Bezugszeichen 131 in Figur 12 kennzeichnet eine Aufnahme eines derartigen Videozentriersystems für die Blickrichtung geradeaus.

Dann wird dem Probanden aufgetragen, ein Fixierziel in einer anderen Blickrichtung anzusehen. Es erfolgt eine erneute Aufnahme des Auges für diese zweite Blickrichtung mit Hilfe des kalibrierten photographischen Systems. Das Bezugszeichen 132 in Figur 12 kennzeichnet eine Aufnahme des Videozentriersystems für die Blickrichtung seitlich. Aus diesen Aufnahmen wird dann die Lage des charakteristischen Augenbestandteils, wie z.B. der Pupille (insbesondere der Pupillenmitte), der Iris, des Limbus, eines Blutgefässes oder dergleichen z.B. mittels eines Computers 133 ermittelt. Aus dem Wissen über die unterschiedlichen Blickrichtungen $\gamma$, d.h. der Bekanntheit des jeweiligen Fixierpunkts, und der jeweiligen Lage des aus den Aufnahmen gewonnenen charakteristischen Augenbestandteils lassen sich jeweils charakteristische Augenachsen, wie z.B. die Fixierlinie FL, und/oder die Sehachse GL und/oder die optische Achse OA für die unterschiedlichen Blickrichtungen $\gamma$ ermitteln. Diese dienen dann zur Ermittlung der Augendrehpunktlage ADL.

Konkret wird beispielsweise in einem ersten Schritt 2a) eine Aufnahme der Lage der Pupille 17 und der Pupillenmitte gemacht, wobei der Proband z.B. geradeaus blickt. Daraus wird die Normale zur Pupillenebene in der Pupillenmitte und damit eine erste Blickrichtung $\gamma_1$ ermittelt.

In einem zweiten Schritt 2b) wird eine Aufnahme der Lage der Pupille 17 und der Pupillenmitte gemacht, wobei der Proband nunmehr seitlich blickt. Daraus wird wiederum die Normale zur Pupillenebene in der Pupillenmitte und damit eine zweite Blickrichtung $\gamma_2$ ermittelt.

In einem dritten Schritt 2c) wird nun aus den beiden unterschiedlichen Blickrichtungen $\gamma_1, \gamma_2$ der Augendrehpunkt Z', d.h. die Augendrehpunktlage ADL, als Schnittpunkt der beiden Normalvektoren ermittelt.

Bei Aufnahmen von mehr als zwei Blickrichtungen $\gamma$ kann auch das bereits erwähnte ausgedehnte, im Allgemeinen näherungsweise kugelförmige Gebiet bestimmt werden, in dem sich die momentanen Augendrehpunkte befinden.

Die zweite Variante, nämlich die Bestimmung der Augendrehpunktlage aus der Form eines charakteristischen Bestandteils des Auges, wird nachfolgend beschrieben:

Wie bei der vorangegangenen Variante erfolgt die Aufnahme des Auges für unterschiedliche Blickrichtungen $\gamma$ mit Hilfe eines kalibrierten photographischen Systems. Aus den Aufnahmen wird dann die Form des charakteristischen Augenbestandteils, wie z.B. der Pupille, der Iris, der Blutgefäße oder dergleichen ermittelt. Aus diesen Formen lassen sich charakteristische Achsen ableiten. Anders als bei der vorstehend beschriebenen Variante ist es bei dieser Verfahrensvariante nicht erforderlich die unterschiedlichen Fixierziele bei den unterschiedlichen Blickrichtungen $\gamma$ zu kennen. Die Achsen dienen dann wiederum zur Ermittlung der Augendrehpunktlage.

Die Augendrehpunktlage wird demgemäß aus einer mathematischen Transformation am Auge 10 bei unterschiedlicher Blickrichtung $\gamma$ bestimmt und für die mathematische Transformation ein kalibriertes photographisches System verwendet.

Drittes Ausführungsbeispiel:

Bei einem dritten Ausführungsbeispiel eines erfindungsgemäßen Verfahrens wird in einem ersten Schritt 3a) die Oberfläche der Hornhaut 14 z.B. bei geradeaus gerichtetem Blick des Probanden dreidimensional gemessen. Hierzu kann man beispielsweise ein Gerät verwenden, wie es von der Anmelderin unter der Bezeichnung "iProfiler" vertrieben wird. Figur 13 skizziert eine Anordnung 140 zur Durchführung des Verfahrens. Der "iProfiler" ist in dieser Figur mit dem Bezugszeichen 141 gekennzeichnet.

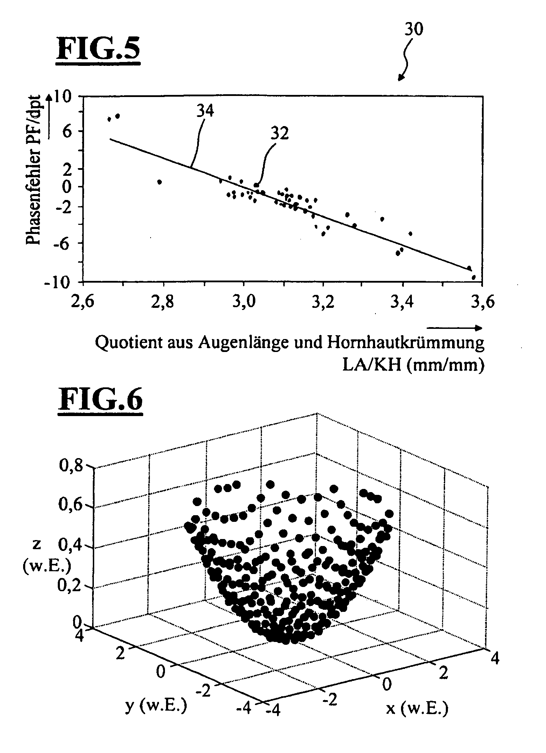

Figur 6 zeigt eine beispielhafte Darstellung der Punkte, die auf der Hornhaut 14 bei geradeaus gerichtetem Blick gemessen werden. Die nach oben gerichtete z-Achse fällt mit dieser Blickrichtung zusammen.

In einem zweiten Schritt 3b) wird die im ersten Schritt ermittelte Oberfläche der Hornhaut 14 z.B. in einem Computer 143 durch eine dreidimensionale mathematische Formel beschrieben.

Zur Ermittlung dieser Formel können die üblichen mathematischen Verfahren eingesetzt werden. Ein Beispiel dafür ist die Approximation bzw. das Fitten an die Punkte durch z.B. Least Squares Fit. Diese Approximation ist zum Beispiel in der Anleitung zur Funktion "Isqnonlin" des Softwarepakets MATLAB der Firma The MathWorks, Inc. beschrieben. Ein weiteres Beispiel sind geeignete mathematische Funktionenklassen, wie Zernike-Polynome und Splines.

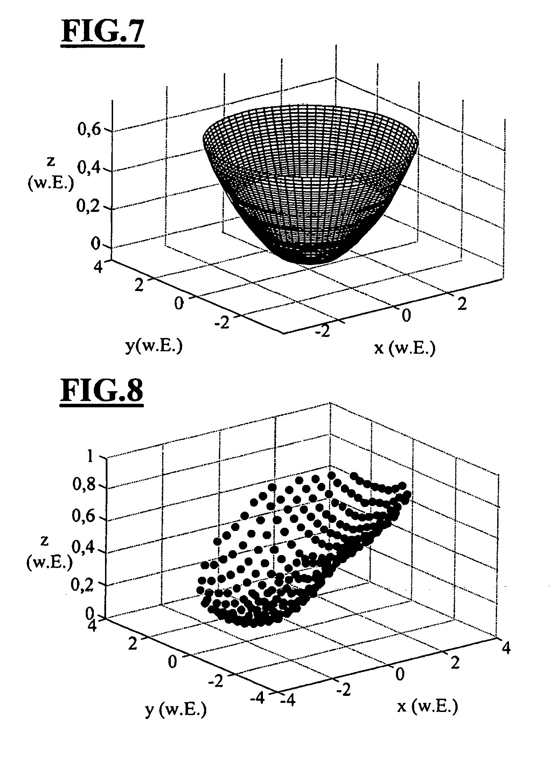

Figur 7 zeigt die Fläche, die durch die in Schritt 3b) erfolgte Approximation an die Hornhautdaten entstanden ist. Für jeden beliebigen Punkt des in Schritt 3b) vermessenen Bereiches der Hornhaut 14 kann jetzt über die zugrunde liegende mathematische Beschreibung ein Punkt auf der Hornhaut 14 berechnet werden.

In einem dritten Schritt 3c) wird mindestens ein Teil der Oberfläche der Hornhaut 14 bei um einen Winkel $\gamma$ geneigten Blick des Probanden in derselben Messposition wie in Schritt 3a) dreidimensional gemessen.

Figur 8 verdeutlicht Schritt 3c). Im dargestellten Ausführungsbeispiel wird für die Messung des Auges 10 bei geänderter Blickrichtung $\gamma$ derselbe Messbereich wie in Schritt 3a) verwendet. Dadurch wandert ein Teil der in Schritt 3a) gemessenen Hornhautfläche aus dem Messbereich heraus, und Teile der Lederhaut 24 wandern in den Messbereich hinein. In Figur 8 wird dies durch den leicht erkennbaren Knick im Verlauf der Messpunkte veranschaulicht.

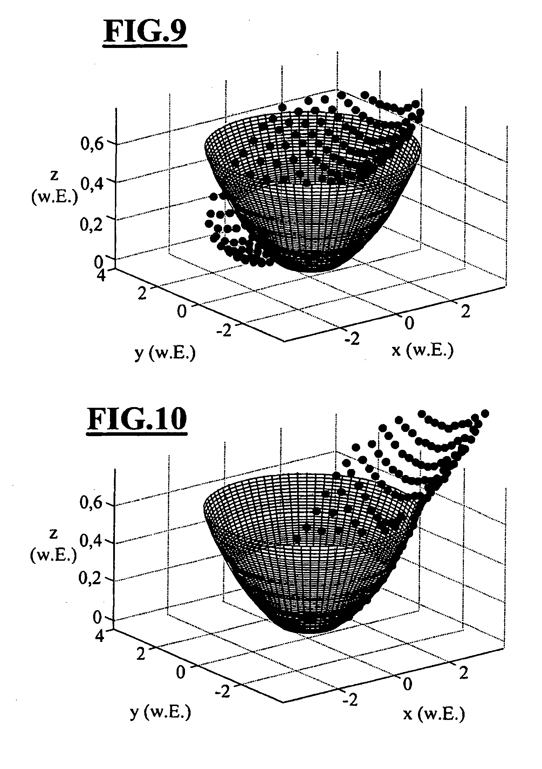

Figur 9 zeigt die Lage der in Schritt 3c) bei geänderter Blickrichtung γ vermessenen Hornhautpunkte im Vergleich zur Lage der Hornhautpunkte in Schritt 3a).

In einem vierten Schritt 3d) werden die Winkel der Blickrichtungen γ sowie die Augendrehpunktlage ADL durch Fitten der in Schritt 3c) ermittelten dreidimensionalen Daten an die in Schritt 3b) ermittelte Formel bestimmt.

Dies geschieht durch den Ansatz einer Drehung um einen Punkt im Raum, der die in Schritt 3c) ermittelten Daten so lange unterworfen werden, bis sie bestmöglich mit der in Schritt 3b) ermittelten mathematischen Beschreibung der Hornhautoberfläche in Übereinstimmung gebracht sind. Die Blickrichtung γ (Vektor) und die Lage des Augendrehpunkts ADL werden bei diesem Verfahren als freie Parameter der Approximation (z.B. Least Squares Fit) an die vorher in Schritt 3b) bestimmte mathematische Beschreibung der Hornhautoberfläche eingesetzt. Nach dem Erreichen des besten Fits sind Augendrehpunktslage ADL und Blickrichtung γ gefunden. Daher ist es in diesem Verfahren unnötig, dem Probanden eine bestimmte Blickrichtung γ vorzuschreiben. Es ist auch ausreichend, in Schritt 3c) nur einen Teil der Hornhautoberfläche zu messen. Wichtig ist nur, dass in Schritt 3c) auch ein Bereich der Hornhaut 14 gemessen wird, der bereits in Schritt 3a) vermessen wurde und in die Ermittlung der mathematischen Formel in Schritt 3b) einbezogen war.

Figur 10 zeigt das Ergebnis der in Schritt 3d) durchgeführten Approximation. Die in Schritt 3c) ermittelten Messpunkte sind durch Drehung um einen Punkt im Raum (Augendrehpunkt) bestmöglich an die in Schritt 3b) gefundene Fläche herangeführt worden. Der im Beispiel zu Schritt 3c), d.h. Figur 8, mitvermessene Teil der Lederhaut 24 liegt an der richtigen Stelle außerhalb der Hornhaut 14.

Viertes Ausführungsbeispiel:

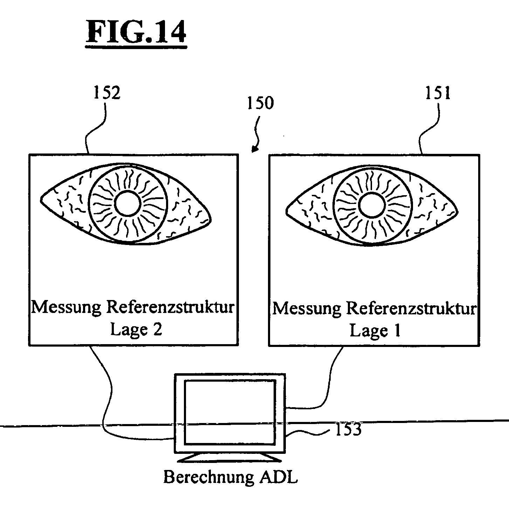

In einem vierten Ausführungsbeispiel eines Verfahrens wird allgemein in einem ersten Schritt 4a) in einer ersten Messsituation eine Referenzstruktur des Auges 10 erfasst. Die entsprechende Anordnung 150 ist in der Figur 14 dargestellt. Die Referenzstruktur ist dabei vorzugsweise eine Struktur der Iris 16 oder eine Struktur von Blutgefäßen in der Lederhaut 24 (vgl. Bezugszeichen 151).

In einem zweiten Schritt 4b) wird die Referenzstruktur des Auges 10 in einer zweiten Messsituation erfasst. Unter der ersten und der zweiten Messsituation ist zu verstehen, dass zwei unterschiedliche Messungen vorgenommen und/oder Messverfahren eingesetzt wurden, vorzugsweise mittels unterschiedlicher Messgeräte 151, 152.

In einem dritten Schritt 4c) wird die Lageveränderung, insbesondere die Verdrehung zwischen den Messsituationen in Schritt 4a) und 4b) rechnerisch (Computer 153) ermittelt und bei der Verordnung des Brillenglases 20 berücksichtigt.

Um in den Schritten 4a) bis 4c) ein gemeinsames Bezugssystem für die gemessenen Lagen der Referenzstrukturen herzustellen, kann erfindungsgemäß in jedem der Schritte 4a) und 4b) eine photographische Aufnahme der Referenzstrukturen hergestellt werden. Diese Referenzstrukturen können dann als gemeinsames Referenzsystem für die Lagen der Pupillenmitte und des Hornhautscheitels sowie weiterer Parameter verwendet werden.

Dieses Verfahren eignet sich z.B. zur Referenzierung der beiden Aufnahmen des Auges bei unterschiedlichen Blickrichtungen, wie diese beim zweiten und dritten Verfahren erforderlich sind.

Bezugszeichenliste

| 10 | Auge |
| 11 | Netzhautgrube |
| 12 | Glaskörper |
| 13 | Netzhautebene |
| 14 | Hornhaut |
| 15 | Hornhautscheitel |
| 16 | Iris |
| 17 | Pupille |
| 18 | Linse |
| 19 | Limbus |
| 20 | Brillenglas |
| 22 | Rückfläche |
| 24 | Lederhaut |
| 30 | Diagramm |
| 32 | Messpunkt |
| 34 | Gerade |

| 41 | Wellenfront |
| 42 | Wellenfront |
| 120 | Vorrichtung zur Bestimmung der Augendrehpunklage |
| 122 | iProfiler |
| 124 | iProfiler |
| 126 | Computer |
| 130 | Vorrichtung zur Bestimmung der Augendrehpunktlage |
| 131 | Videozentriergerät (Aufnahme 1) |
| 132 | Videozentriergerät (Aufnahme 2) |
| 133 | Computer |
| 140 | Vorrichtung zur Bestimmung der Augendrehpunktlage |
| 141 | iProfiler |
| 143 | Computer |
| 150 | Vorrichtung zur Bestimmung von mindestens 2 optischen Parametern |
| 151 | Videozentriergerät (Aufnahme 1) |
| 152 | Videozentriergerät (Aufnahme 2) |
| 153 | Computer |

| ADL | Augendrehpunktlage |
| HA | objektseitiger Hauptpunkt des Auges |
| HSA | Hornhaut-Scheitelabstand |
| FL | Fixierlinie |
| GL | Sehachse |
| PF | mittlerer Phasenfehler |
| KH | Krümmung der Hornhaut |
| LA | Augenlänge |
| M | mechanischer Augendrehpunkt |
| OA | optische Achse |
| Z' | optischer Augendrehpunkt |
| x, y, z | Raumkoordinaten |

$\gamma$  Winkel,Blickrichtung

## Patentansprüche

1. Verfahren zur Bestimmung der Augendrehpunktlage (ADL) in einem Auge (10) eines Probanden, **gekennzeichnet durch** die Schritte:

   a) dreidimensionales Messen wenigstens eines Teils der Oberfläche der Hornhaut (14) des Auges (10) bei einer ersten Blickrichtung des Probanden;
   b) Beschreiben der in Schritt a) ermittelten Oberfläche der Hornhaut (14) **durch** eine dreidimensionale mathematische Formel;
   c) dreidimensionales Messen mindestens eines Teils der Oberfläche der Hornhaut (14) bei gegenüber der Blickrichtung in Schritt a) geänderter Blickrichtung des Probanden in derselben Messposition wie bei Schritt a), bei gegenüber Schritt a) unveränderter Kopfposition des Probanden;
   d) Bestimmen der Blickrichtung in Schritt c) und/oder der Augendrehpunktlage (ADL) **durch** Fitten der in Schritt c) gemessenen dreidimensionalen Daten an die in Schritt b) ermittelte Formel, wobei eine mathematische Transformation, insbesondere Drehung um einen Punkt im Raum, verwendet wird.

2. Verfahren zum Optimieren eines für ein Auge (10) eines Probanden individuellen Brillenglases (20), bei dem die Augendrehpunktlage (ADL) ermittelt und als Eingangsparameter verwendet wird, **dadurch gekennzeichnet, dass** zur Bestimmung der Augendrehpunktlage (ADL) in dem Auge (10) des Probanden ein Verfahren nach Anspruch 1 verwendet wird.

3. Computerprogramm mit Programmcode eingerichtet zur Durchführung des Verfahrens nach einem der vorangegangenen Ansprüche, wenn das Programm in einer Vorrichtung gemäß Anspruch 5 ausgeführt wird.

**4.** Computerprogramm nach Anspruch 3, gespeichert auf einem maschinenlesbaren Datenträger.

**5.** Vorrichtung (140) zur Bestimmung der Augendrehpunktlage (ADL) in einem Auge (10) eines Probanden, **gekennzeichnet durch**:

a) eine Messeinrichtung (141) zum dreidimensionalen Messen wenigstens eines Teils der Oberfläche der Hornhaut (14) des Auges (10) bei einer ersten Blickrichtung des Probanden und zum dreidimensionalen Messen mindestens eines Teils der Oberfläche der Hornhaut (14) bei gegenüber der ersten Blickrichtung geänderter zweiter Blickrichtung des Probanden in derselben Messposition und bei unveränderter Kopfposition des Probanden;

b) eine Recheneinrichtung (143) zum Beschreiben der zur ersten Blickrichtung ermittelten Oberfläche der Hornhaut (14) **durch** eine dreidimensionale mathematische Formel und zum Bestimmen der zweiten Blickrichtung sowie der Augendrehpunktlage (ADL) **durch** Fitten der zur zweiten Blickrichtung gemessenen dreidimensionalen Daten an die zur ersten Blickrichtung ermittelte Formel, wobei eine mathematische Transformation, insbesondere Drehung um einen Punkt im Raum, verwendet wird.

**Claims**

**1.** Method for determining the location of the eye fulcrum (ADL) in an eye (10) of a test subject, **characterized by** the steps of:

a) measuring in three dimensions at least a portion of the surface of the cornea (14) of the eye (10) for a first viewing direction of the test subject;

b) describing the surface of the cornea (14) determined in step a) by a three-dimensional mathematical formula;

c) measuring in three dimensions at least a portion of the surface of the cornea (14) for a viewing direction of the test subject altered by comparison with the viewing direction in step a), doing so at the same measurement position as for step a), for a head position of the test subject unchanged by comparison with step a); and

d) determining the viewing direction in step c) and/or the location of the eye fulcrum (ADL) by fitting the three-dimensional data measured in step c) to the formula determined in step b), use being made of a mathematical transformation, in particular rotation about a point in space.

**2.** Method for optimizing a spectacle lens (20) customized for an eye (10) of a test subject, in which the location of the eye fulcrum (ADL) is determined and used as input parameter, **characterized in that** a method according to Claim 1 is used in determining the location of the eye fulcrum (ADL) in the eye (10) of the test subject.

**3.** Computer program with program code set up to carry out the method according to either of the preceding claims when the program is executed in an apparatus according to Claim 5.

**4.** Computer program according to Claim 3, stored on a machine-readable data medium.

**5.** Apparatus (140) for determining the location of the eye fulcrum (ADL) in an eye (10) of a test subject, **characterized by**:

a) a measurement device (141) for measuring in three dimensions at least a portion of the surface of the cornea (14) of the eye (10) for a first viewing direction of the test subject, and for measuring in three dimensions at least a portion of the surface of the cornea (14) for a second viewing direction of the test subject altered by comparison with the first viewing direction at the same measurement position and for an unchanged head position of the test subject; and

b) a computing device (143) for describing the surface of the cornea (14) determined in relation to the first viewing direction by a three-dimensional mathematical formula, and for determining the second viewing direction as well as the location of the eye fulcrum (ADL) by fitting the measured three-dimensional data relating to the second viewing direction to the formula determined in relation to the first viewing direction, use being made of a mathematical transformation, in particular rotation about a point in space.

**Revendications**

1. Procédé de détermination de la position du point de rotation d'oeil (ADL) dans un oeil (10) d'un sujet d'étude, **caractérisé par** les étapes suivantes :

   a) mesure tridimensionnelle d'au moins une partie de la surface de la cornée (14) de l'oeil (10) avec une première direction du regard du sujet d'étude ;
   b) description de la surface de la cornée (14) déterminée à l'étape a) par une formule mathématique tridimensionnelle ;
   c) mesure tridimensionnelle d'au moins une partie de la surface de la cornée (14) avec une direction du regard du sujet d'étude modifiée par rapport à la direction du regard dans l'étape a) dans la même position de mesure qu'à l'étape a), avec une position de la tête du sujet d'étude inchangée par rapport à l'étape a) ;
   d) détermination de la direction du regard dans l'étape c) et/ou de la position du point de rotation d'oeil (ADL) par adaptation des données tridimensionnelles mesurées dans l'étape c) à la formule déterminée dans l'étape b), une transformation mathématique, notamment une rotation autour d'un point dans l'espace, étant utilisée.

2. Procédé d'optimisation d'un verre de lunette (20) personnalisé pour un oeil (10) d'un sujet d'étude, selon lequel la position du point de rotation d'oeil (ADL) est déterminée et utilisée comme paramètre d'entrée, **caractérisé en ce qu'**un procédé selon la revendication 1 est utilisé pour déterminer la position du point de rotation d'oeil (ADL) dans l'oeil (10) du sujet d'étude.

3. Programme informatique comprenant un code de programme conçu pour mettre en oeuvre le procédé selon l'une des revendications précédentes lorsque le programme est exécuté dans un dispositif selon la revendication 5.

4. Programme informatique selon la revendication 3, enregistré sur un support de données lisible par machine.

5. Dispositif (140) pour déterminer la position du point de rotation d'oeil (ADL) dans un oeil (10) d'un sujet d'étude, **caractérisé par** :

   a) un équipement de mesure (141) pour la mesure tridimensionnelle d'au moins une partie de la surface de la cornée (14) de l'oeil (10) avec une première direction du regard du sujet d'étude et pour la mesure tridimensionnelle d'au moins une partie de la surface de la cornée (14) avec une direction du regard du sujet d'étude modifiée par rapport à la première direction du regard dans la même position de mesure et avec une position de la tête du sujet d'étude inchangée ;
   b) un équipement de calcul (143) pour décrire la surface de la cornée (14) déterminée en rapport avec la première direction du regard par une formule mathématique tridimensionnelle et pour déterminer la deuxième direction du regard ainsi que la position du point de rotation d'oeil (ADL) par adaptation des données tridimensionnelles mesurées en rapport avec la deuxième direction du regard à la formule déterminée en rapport avec la première direction du regard, une transformation mathématique, notamment une rotation autour d'un point dans l'espace, étant utilisée.

## FIG.1

## FIG.2

## FIG.3

## FIG.4

## FIG.5

30

Phasenfehler PF/dpt

34

32

10
6
2
0
-2
-6
-10

2,6    2,8    3,0    3,2    3,4    3,6

Quotient aus Augenlänge und Hornhautkrümmung
LA/KH (mm/mm)

## FIG.6

z
(w.E.)

0,8
0,6
0,4
0,2
0

4
2
0
-2
-4

-4    -2    0    2    4

y (w.E.)

x (w.E.)

## FIG.7

## FIG.8

## FIG.9

## FIG.10

# FIG.11

120

122

124

126

Bestimmung KH

Bestimmung PF

Berechnung ADL aus LA

## FIG.12

Messung Blick seitlich

Messung Blick geradeaus

Berechnung ADL

## FIG.13

# FIG.14

152

150

151

Messung Referenzstruktur
Lage 2

Messung Referenzstruktur
Lage 1

153

Berechnung ADL

**IN DER BESCHREIBUNG AUFGEFÜHRTE DOKUMENTE**

**In der Beschreibung aufgeführte Patentdokumente**

- EP 857993 B1
- EP 562336 B1
- WO 2006106248 A1
- EP 0825826 A1